# EUROPÄISCHE PATENTSCHRIFT

(11) **EP 1 562 529 B1**
(45) Veröffentlichungstag und Bekanntmachung des Hinweises auf die Patenterteilung: **10.05.2006**
(21) Anmeldenummer: 03748095.1
(22) Anmeldetag: 26.09.2003
(51) Int. Cl.: A61G 13/12, A61G 7/07, A47C 31/00

(54) **KOPFSTÜTZE FÜR PATIENTENLAGERFLÄCHE**
HEADREST FOR A PATIENT-BEARING SURFACE
APPUI-TETE POUR UNE SURFACE DE SUPPORT POUR PATIENTS

(30) Priorität: 18.11.2002 DE 20217825 U
(43) Veröffentlichungstag der Anmeldung: 17.08.2005
(73) Patentinhaber: MAQUET GmbH & Co. KG, 76437 Rastatt (DE)
(72) Erfinder: PIONTEK, Manfred, 76185 Karlsruhe (DE)
(74) Vertreter: Schaumburg, Thoenes, Thurn, Landskron
(86) Internationale Anmeldenummer: PCT/EP2003/010719
(87) Internationale Veröffentlichungsnummer: WO 2004/045481

(56) Entgegenhaltungen:
- WO-A-00/47155
- WO-A-01/76403
- WO-A-97/25956
- US-B1- 6 374 441

## Beschreibung

Die Erfindung betrifft eine Kopfstütze für eine Patientenlagerfläche, insbesondere an Operationstischen, gemäß dem Oberbegriff des Anspruchs,1.

Aus der US 6,276,012 B2 ist eine Kopfstütze bekannt, die aus einem U-förmigen Teil und einem zwischen den U-Schenkeln liegenden plattenförmigen Abschnitt besteht. In der Rückenlage wird der Kopf des Patienten von beiden Teilen unterstützt. In der Bauchlage wird das plattenförmige Teil weggeklappt, so dass der Kopf des Patienten mit der Stirn auf dem Mittelsteg des U aufliegt und zumindest Mund und Nase des Patienten frei liegen. Die Kopfstütze insgesamt ist im wesentlichen eben ausgebildet und gepolstert. Sowohl in der Rückenlage als auch in der Bauchlage ist der Kopf seitlich abgestützt.

Aus der US-A-6,042,184 ist eine Ruheliege bekannt, die mit einer plattenförmigen Kopfstütze versehen ist. In der plattenförmigen Kopfstütze ist eine Öffnung ausgebildet, die von einem kreisringförmigen, nicht ganz geschlossenen. Polster umgeben ist, das auf der Platte beispielsweise mit Druckknöpfen befestigt werden kann. Auch hier kann der Kopf einer Person nicht nur in der Rückenlage sondern auch in der Bauchlage abgestützt werden, wobei das Gesicht frei liegt. Die Kopfstütze ist jedoch nicht ausreichend an die menschliche Kopfform angepasst und ungeeignet, den Kopf eines Patienten während einer Operation, bei welcher der Patient längere Zeit regungslos gehalten werden muss, so abzustützen, dass der Patient keinen Schaden nimmt, beispielweise durch Druckstellen oder dergleichen.

Die WO 01/76403 A1 zeigt eine Kopfstütze, umfassend eine helmartig gewölbte Stützschale mit Öffnungen für die Augenpartie, Mund und Nase und ein Schaumstoffpolster mit entsprechenden Öffnungen zur-Abstützung des Gesichts.

Die US 6,374,441 B1 zeigt eine Kopfstütze zur Abstützung des Gesichtes eines Patienten mit einem Brett, auf dem ein Schaumstoffpolster angeordnet ist, in das eine Kontur mit einem Hohlraum für die Augenpartie, Mund und Nase eingeschnitten ist.

Der Erfindung liegt die Aufgabe zugrunde, eine Kopfstütze der eingangs genannten Art anzugeben, die es ermöglicht, den Kopf in einer gewünschten Position sowohl in der Rückenlage als auch der Bauchlage des Patienten sicher für den Patienten schonend zu unterstützen.

Zur Lösung dieser Aufgabe hat die Stützschale der erfindungsgemäßen Kopfstütze eine annähernd hufeisenförmige Gestalt mit einem zum Unterstützen des Hinterkopfes oder der Stirn bestimmten zentralen Abschnitt, dessen Auflagefläche mindestens annähernd kugelschalenförmig ist , und mit zwei einen Abstand voneinander aufweisenden Seitenabschnitten, deren Auflageflächen mindestens annähernd einer gemeinsamen Zylinderfläche angepasst sind, deren Achse parallel zu einer zwischen den Seitenabschnitten verlaufenden Symmetrielinie der Kopfstütze ist, wobei an den Seitenabschnitten jeweils eine in Richtung auf den jeweils anderen Seitenabschnitt vorspringende Jochbeinstütze ausgebildet ist.

Die erfindungsgemäße Kopfstütze ist an die spezielle Form des menschlichen Kopfes angepasst und unterstützt den Kopf an den für eine solche Unterstützung geeigneten Flächen des Schädels, nämlich dem Hinterkopf oder der Stirn sowie den Wangenknochen. Durch die kugelschalenförmige bzw. zylindrische Wölbung der Abschnitte ist der Kopf so gebettet, dass er nicht nach der Seite umfallen kann. Die Jochbeinstützen an den Seitenabschnitten ermöglichen einerseits eine gute Unterstützung des Gesichtes in der Bauchlage des Patienten, wobei dennoch die Augenpartie sowie Mund und Nase für das Atmen bzw. die Beatmung sowie das Ansetzen einer Narkosemaske oder anderer Hilfsmittel frei belieben. Durch die anatomisch gerechte Form der erfindungsgemäßen Kopfstütze wird der Auflagedruck des Kopfes großflächiger über die Abschnitte der Kopfstütze verteilt, so dass die lokale Belastung vermindert wird und damit auch die längeren Operationen Druckstellen am Kopf, insbesondere im Gesicht vermieden werden können.

Im Gegensatz zu herkömmlichen Kopfstützen, bei denen zur seitlichen Abstützung des Kopfes das Polster sehr dick gemacht werden muss, d.h. der Kopf relativ weit in das Polster einsinkt, kann bei der erfindungsgemäßen Lösung wegen der anatomisch korrekten Form der Kopfstütze das Auflagepolster dünner gemacht werden. Die Stützschale ist zweckmäßigerweise aus Kunststoff hergestellt, was sowohl die Herstellung beispielsweise im Spritzgußverfahren als auch die Pflege der Stützschale im täglichen Betrieb erleichtert.

Vorzugsweise hat das Auflagepolster an einer der Stützschale zugewandten Seite mindestens zwei Steckstifte, die zum Eingriff in die Stützschale durchsetzende Bohrungen bestimmt sind. Auf diese Weise kann das Auflagepolster schnell und sicher mit der Stützschale verbunden und wieder von dieser gelöst werden. Sowohl die Bohrungen als auch die Steckstifte sind einfach und sicher zu reinigen. Um ein ungewolltes Lösen des Auflagepolsters von der Stützschale zu vermeiden, kann es zweckmäßig sein, wenn die Steckstifte an einem zylindrischen Schaft jeweils einen elastisch nachgiebigen Bund haben, dessen Durchmesser gegenüber dem Bohrungsdurchmesser geringfügig größer ist, so daß die Steckstifte nur mit einem gewissen Widerstand durch die Bohrungen in der Stützschale gedrückt bzw. herausgezogen werden können.

Die erfindungsgemäße Kopfstütze kann einteilig ausgebildet sein oder auch entlang ihrer Symmetrielinie in zwei spiegelbildlich gleiche Teilstützen unterteilt sein. Letztere Ausführungsform ermöglicht eine gewisse Anpassung der Kopfstütze an unterschiedlich große Köpfe.

Zur Verbindung der Kopfstütze mit der Patientenlagerfläche kann die Kopfstütze bzw. jede Teilstütze in an sich bekannter Weise mit einem Befestigungskloben zur Halterung an einer Profilschiene verbunden sein, die ihrerseits an einer Patientenlagerfläche oder einer mit dieser verbundenen Halterung befestigt ist. Um sicherzustellen, daß der Patient während einer Operation den Kopf nicht bewegt, kann an dem Außenrand jedes Seitenabschnittes eine Öse zur Befestigung eines Bandes ausgebildet sein, mit dem der Patientenkopf auf der Kopfstütze fixiert werden kann.

Weitere Merkmale und Vorteile der Erfindung ergeben sich aus der folgenden Beschreibung, welche in Verbindung mit den beigefügten Zeichnungen die Erfindung anhand von Ausführungsbeispielen erläutert. Es zeigen:
- Figur 1: eine Draufsicht auf die Stützschale einer ersten Ausführungsform der erfindungsgemäßen Kopfstütze,
- Figur 2: eine Seitenansicht einer mit einem Auflagepolster und einem Fixierband versehenen Kopfstütze,
- Figur 3: eine perspektivische Unteransicht eines für eine Stützschale gemäß Figur 1 bestimmten Auflagepolsters,
- Figur 4: einen Teilschnitt durch die Stützschale entlang Linie IV-IV in Figur 1 und
- Figur 5: eine der Figur 1 entsprechende Draufsicht auf eine Stützschale einer zweiten Ausführungsform der erfindungsgemäßen Kopfstütze.

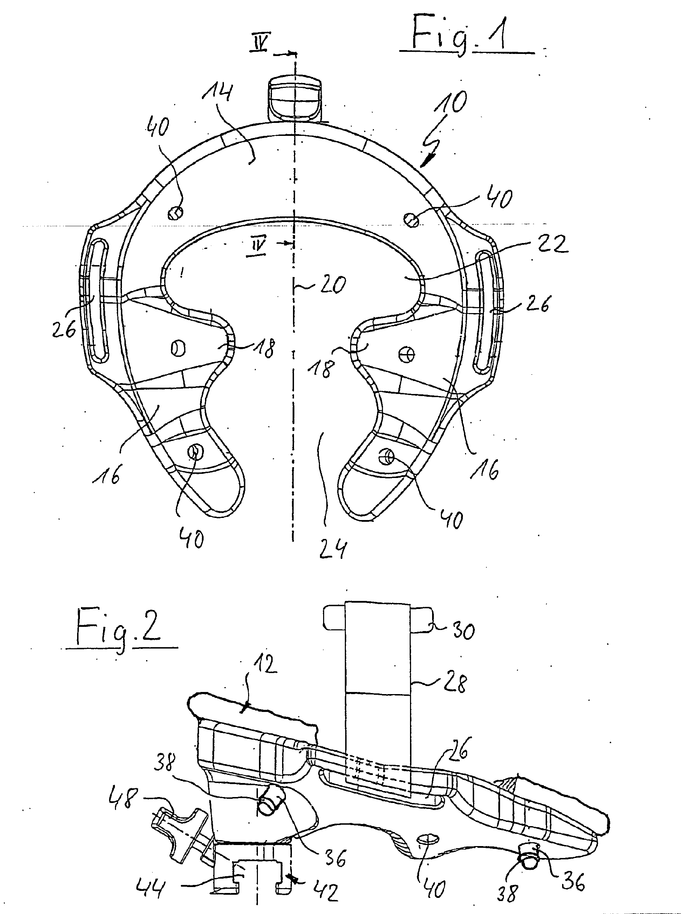

Die in den Figuren 1 bis 4 dargestellte Kopfstütze hat eine im wesentlichen hufeisenförmige Gestalt und umfaßt eine allgemein mit 10 bezeichnete Stützschale und ein dieser Stützschale in seiner Form angepaßtes Auflagepolster 12. Die Stützschale hat einen zentralen Abschnitt 14, der zur Unterstützung des Hinterkopfes eines Patienten (Rückenlage) oder der Stirn (Bauchlage) bestimmt ist. Dieser mittlere Abschnitt 14 ist auf seiner dem Betrachter der Figur 1 zugewandten Ober- oder Innenseite mindestens annähernd kugelschalenförmig ausgebildet. An den zentralen Abschnitt 14 schließen sich zwei Seitenabschnitte 16 an, die sich mit ihren freien Enden einander annähern und jeweils einen in Richtung auf den jeweils anderen Seitenabschnitt vorspringenden Abschnitt 18 haben, der eine Jochbeinstütze bildet, d.h. das Gesicht eines auf dem Bauch liegenden Patienten im Bereich der Wangenknochen unterstützt. Die Seitenabschnitte 16 sind annähernd an eine gemeinsame Zylinderfläche angepaßt, deren Achse parallel zu der zwischen den beiden Seitenabschnitten 16 verlaufenden Symmetrieachse 20 der Kopfstütze verläuft. Die zentrale Aussparung der Stützschale 10 umfaßt einen dem zentralen Abschnitt 14 nahen Bereich 22 entsprechend der Augenpartie des Patientengesichtes und einen der Mundpartie des Patientengesichtes entsprechenden Bereich 24. Durch die Anpassung der Stützschale 14 an die anatomische Form des Kopfes wird einerseits eine bessere seitliche Abstützung und andererseits eine großflächigere Auflage des Kopfes auf der Kopfstütze erreicht, wodurch wiederum die lokale Druckbelastung und damit die Gefahr der Bildung von Druckstellen am Kopf des Patienten vermindert wird.

An den Außenrändern der Seitenabschnitte 16 ist jeweils eine Öse 26 vorgesehen, durch die ein Band 28 gezogen werden kann, mit dem der Kopf eines Patienten auf der Kopfstütze fixiert werden kann. Das Band 28 wird dabei durch die Öse 26 durchgezogen, wonach die Enden des Bandes umgeschlagen und beispielsweise mittels eines Klettverschlusses festgelegt werden, wie dies in Figur 2 angedeutet ist. Auf der Innenseite des Bandes befindet sich ein Polster 30 über das das Band 28 an dem Kopf des Patienten anliegt.

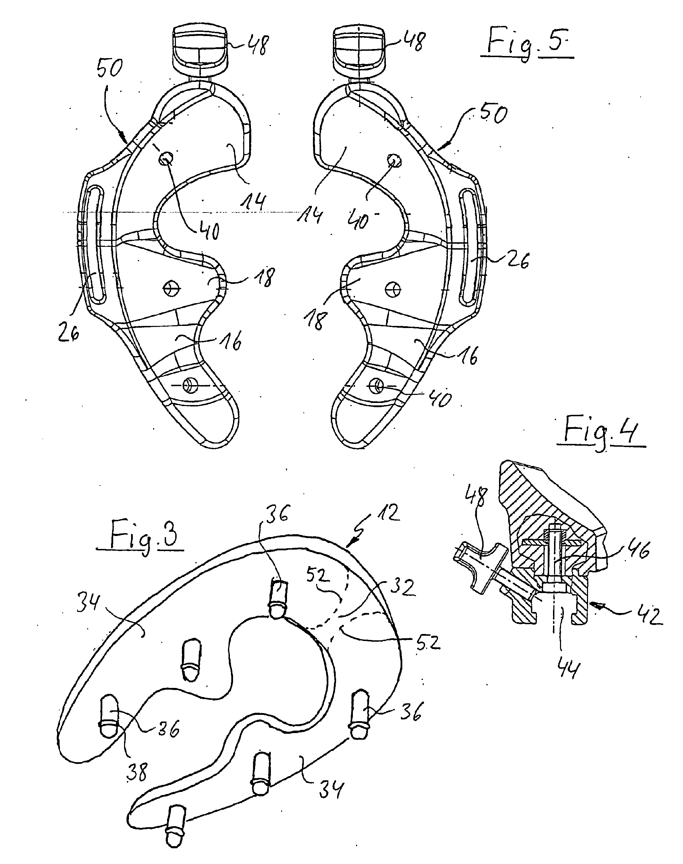

Das mit der Stützschale gemäß Figur 1 zu verbindende Auflagepolster ist in Figur 3 dargestellt. Es hat eine der Form der Stützschale 10 angepaßte Gestalt mit einem zentralen Abschnitt 32 und Seitenabschnitten 34. An seiner der Stützschale 10 zugewandten Seite trägt das Auflagepolster Steckstifte 36, die im wesentlichen zylindrisch ausgebildet sind und in einer umlaufenden Nut einen O-Ring 38 tragen. Die Steckstifte 36 werden in in der Stützschale 10 vorgesehene Bohrungen 40 gesteckt, wobei die Abmessungen der Bohrungen 40 und der Steckstifte 36 so gewählt sind, daß die O-Ringe dem Einstecken und Herausziehen der Steckstifte 36 in die bzw. aus den Bohrungen 40 einen gewissen Widerstand entgegensetzen. Das Auflagepolster 12 kann aber auf diese Weise rasch mit der Stützschale 10 verbunden bzw. von dieser gelöst werden, so daß die Stützschale 10 und das Auflagepolster 12 problemlos und hygienisch einwandfrei gereinigt werden können.

Figur 4 zeigt einen mit der Stützschale verbundenen Befestigungskloben 42, der eine Profilöffnung 44 hat, mit der er auf eine Profilschiene aufschiebbar ist und der mittels einer Schraube 46 an der Stützschale 10 befestigt ist. Mittels einer Klemmschraube 48 kann der Kloben 42 und damit die gesamte Kopfstütze auf der Profilschiene festgelegt werden.

Figur 5 zeigt eine zweiteilige Stützschale für eine zweiteilige Kopfstütze. Die beiden Teilschalen 50 sind bezüglich der Symmetrielinie 20 spiegelsymmetrisch zueinander ausgebildet und entsprechen in allen Einzelheiten den entsprechenden Abschnitten der einteiligen Stützschale gemäß Figur 1 mit der Ausnahme, daß jede Teilschale 50 mit einem Befestigungskloben 42 versehen ist. Das zugehörige Auflagepolster ist ebenso unterteilt wie die Stützschale, wie dies in Figur 3 durch die gestrichelten Linien 52 angedeutet ist. Die Ausführungsform gemäß Figur 5 bietet die Möglichkeit, durch Veränderung des Abstandes zwischen den beiden Teilstützen die Kopfstütze an die Größe des abzustützenden-Kopfes anzupassen.

## Patentansprüche

1. Kopfstütze für eine Patientenlagerfläche mit einer starren Stützschale (10) und einem mit dieser lösbar verbindbaren Auflagepolster (12), **dadurch gekennzeichnet, dass** die Stützschale (10) eine annähernd hufeisenförmige Gestalt hat mit einem zum Unterstützen des Hinterkopfes oder der Stirn bestimmten zentralen Abschnitt (14), dessen Auflagefläche mindestens annähernd kugelschalenförmig ist, und mit zwei einen Abstand voneinander aufweisenden Seitenabschnitten (16), deren Auflageflächen mindestens annähernd einer gemeinsamen Zylinderfläche angepasst sind, deren Achse parallel zu einer zwischen den Seitenabschnitten (16) verlaufenden Symmetrielinie (20) der Kopfstütze ist, wobei an den Seitenabschnitten (16) jeweils eine in Richtung auf den jeweils anderen Seitenabschnitt (16) vorspringende Jochbeinstütze (18) ausgebildet ist.

2. Kopfstütze nach Anspruch 1, **dadurch gekennzeichnet, dass** die Stützschale (10) aus Kunststoff besteht.

3. Kopfstütze nach Anspruch 1 oder 2, **dadurch gekennzeichnet, dass** das Auf lagepolster (12) an seiner der Stützschale zugewandten Seite mindestens zwei Steckstifte (36) trägt, die zum Eingriff in die Stützschale (10) durchsetzende Bohrungen (40) bestimmt sind.

4. Kopfstütze nach Anspruch 3, **dadurch gekennzeichnet, dass** die Steckstifte (36) jeweils einen zylindrischen Schaft haben, der einen elastisch nachgiebigen Bund (38) mit einem gegenüber dem Bohrungsdurchmesser geringfügig größeren Außendurchmesser hat.

5. Kopfstütze nach einem der Ansprüche 1 bis 4, **dadurch gekennzeichnet, dass** sie entlang ihrer Symmetrielinie (20) in zwei spiegelbildlich gleiche Teilstützen (50) unterteilt ist.

6. Kopfstütze nach einem der Ansprüche 1 bis 5, **dadurch gekennzeichnet, dass** die Kopfstütze bzw. jede Teilstütze mit einem Befestigungskloben (42) zu ihrer Halterung an einer Profilschiene verbunden ist.

7. Kopfstütze nach einem der Ansprüche 1 bis 6, **dadurch gekennzeichnet, dass** an dem Außenrand jedes Seitenabschnittes (16) eine Öse (26) zur Befestigung eines zum Fixieren des Patientenkopfes auf der Kopfstütze bestimmten Bandes ausgebildet ist.

## Claims

1. Headrest for a patient-bearing surface with a rigid support shell (10) and a support padding (12) which is adapted to be connected thereto in a releasable way, **characterised in that** the support shell (10) has a virtually horseshoe-shaped form with a central section (14) for supporting the back of the head or the forehead, the support area of the central section (14) being formed virtually as a spherical shell, and with two side sections (16) spaced apart from each other whose support areas are adapted at least virtually to a common cylinder area whose axis is parallel to a symmetry line (20) of the headrest running between the side sections (16), wherein a cheek-bone support (18) is formed on each of the side sections (16) projecting in the direction of the other side section (16).

2. Headrest according to claim 1, **characterised in that** the support shell (10) is made of plastic.

3. Headrest according to claim 1 or 2, **characterised in that** the support padding (12) has at least two insert pins (36) on its side facing the support shell which are intended for insertion into bores (40) going through the support shell (10).

4. Headrest according to claim 3, **characterised in that** the insert pins (36) each have a cylindrical shaft which has an elastically yielding collar (38) with an outer diameter which is slightly larger than the bore diameter.

5. Headrest according to one of the claims 1 to 4, **characterised in that** it is sub-divided along its symmetry line (20) into two equal mirror-symmetrical sub-supports (50).

6. Headrest according to one of the claims 1 to 5, **characterised in that** the headrest or each sub-support is connected to a fixing hook (42) for the purpose of holding it on a profile track.

7. Headrest according to one of the claims 1 to 6, **characterised in that** on the outer edge of each side section (16) an eye (26) is formed for securing a strap intended for the fixing of the head of the patient on the headrest.

## Revendications

1. Appui-tête pour surface de support de patient, comportant une coque d'appui rigide (10) et un coussin d'appui (12) pouvant être relié de façon amovible à cette coque d'appui, **caractérisé en ce que** la coque d' appui (10) a à peu près une forme de fer à cheval, avec une portion centrale (14) qui est destinée à soutenir l'occiput ou le front et dont la surface de support à au moins approximativement une forme de cuvette, et deux portions latérales (16) qui sont distantes l'une de l'autre et dont les surfaces de support sont adaptées au moins approximativement à une surface cylindrique commune dont l'axe est parallèle à un axe de symétrie (20), s'étendant entre les portions latérales (16), de l'appui-tête, un support de pommette (18) étant conformé au niveau de chacune des portions latérales (16) et saillant en direction de la portion latérale opposée (16).

2. Appui-tête selon la revendication 1, **caractérisé en ce que** la coque d'appui (10) est en matière plastique.

3. Appui-tête selon la revendication 1 ou 2, **caractérisé en ce que** le coussin d'appui (12) supporte, du côté opposé à la coque d'appui, au moins deux doigts enfichables (36) qui sont destinés à s'accrocher dans des perçages (40) traversant la coque d'appui (10).

4. Appui-tête selon la revendication 3, **caractérisé en ce que** les doigts enfichables (36) comportent chacun un axe cylindrique qui présente un bourrelet (38) flexible élastiquement dont le diamètre extérieur est légèrement supérieur au diamètre du perçage.

5. Appui-tête selon l'une des revendications 1 à 4, **caractérisé en ce qu'**il est divisé, le long de sa ligne de symétrie (1), en deux sections d'appui-tête (50) de forme identique en image miroir.

6. Appui-tête selon l'une des revendications 1 à 5, **caractérisé en ce que** l'appui-tête, respectivement chaque section d'appui-tête, est relié à un étau de fixation (42) destiné à maintenir celui-ci ou celle-ci sur un rail profilé.

7. Appui-tête selon l'une des revendications 1 à 6, **caractérisé en ce qu'**un oeillet (26) est conformé au niveau du bord extérieur de chaque portion latérale (16) en vue de fixer une bande destinée à immobiliser la tête du patient sur l'appui-tête.
